# EUROPEAN PATENT SPECIFICATION

(11) **EP 2 964 755 B1**
(45) Date of publication and mention of the grant of the patent: **01.11.2017**
(21) Application number: 13877167.0
(22) Date of filing: 08.03.2013
(51) Int. Cl.: C12N 5/0787, C12N 5/0789, C12N 5/0775

(54) **MENSTRUAL STEMS CELLS FOR THE EFFICIENT SUPPORT AND EXPANSION OF CD34+ CD133+ HEMATOPOIETIC STEM CELLS IN VITRO**
MENSTRUELLE STAMMZELLEN ZUR UNTERSTÜTZUNG UND ERWEITERUNG VON HÄMATOPOIETISCHEN CD34+CD133+- STAMMZELLEN IN VITRO
CELLULES SOUCHES DE SANG MENSTRUEL POUR LE SOUTIEN ET LE DÉVELOPPEMENT EFFICACES DES CELLULES SOUCHES HÉMATOPOÏÉTIQUES CD34+ ET CD133+ IN VITRO

(43) Date of publication of application: 13.01.2016
(73) Proprietor: Cells for Cells, Santiago (CL)
(72) Inventor: KHOURY, Maroun, Santiago (CL); ALCAYAGA-MIRANDA,Francisca, Santiago (CL)
(74) Representative: Lawrence, John
(86) International application number: PCT/IB2013/051861
(87) International publication number: WO 2014/135924

(56) References cited:
- WO-A1-2005/124350
- WO-A1-2009/058365
- WO-A1-2010/138873
- WO-A1-2013/007284
- WO-A1-2013/025189
- WO-A2-2008/109063
- US-A- 5 843 633
- US-A1- 2004 143 863
- US-A1- 2009 191 628
- US-A1- 2011 306 516
- MENG XIAOLONG ET AL: "Endometrial regenerative cells: A novel stem cell population", JOURNAL OF TRANSLATIONAL MEDICINE, BIOMED CENTRAL, LONDON, GB, vol. 5, no. 1, 15 November 2007 (2007-11-15), page 57, XP021037640, ISSN: 1479-5876
- RODRÍGUEZ-PARDO VIVIANA M ET AL: "Mesenchymal stem cells promote a primitive phenotype CD34+c-kit+ in human cord blood-derived hematopoietic stem cells duringex vivoexpansion", CELLULAR & MOLECULAR BIOLOGY LETTERS, UNIVERSITY OF WROCAW. INSTITUTE OF BIOCHEMISTRY, WROCAW; PL, PL, vol. 18, no. 1, 27 October 2012 (2012-10-27), pages 11-33, XP035994748, ISSN: 1425-8153, DOI: 10.2478/S11658-012-0036-1 [retrieved on 2012-10-27]
- FRANCISCA ALCAYAGA-MIRANDA ET AL: "Characterization of menstrual stem cells: angiogenic effect, migration and hematopoietic stem cell support in comparison with bone marrow mesenchymal stem cells", STEM CELL RESEARCH & THERAPY, BIOMED CENTRAL LTD, LONDON, UK, vol. 6, no. 1, 17 March 2015 (2015-03-17), page 32, XP021215844, ISSN: 1757-6512, DOI: 10.1186/S13287-015-0013-5
- MENG ET AL.: 'Endometrial regenerative cells: A novel stem cell population.' JOURNAL OF TRANSLATIONAL MEDICINE vol. 5, no. 57, 15 November 2007, pages 1 - 10, XP021037640

## Description

### FIELD OF THE INVENTION

The invention relates to highly proliferative stem cell obtained from menstrual fluid. The menstrual stem cells (MenSCs) may be cultured in vitro and exhibit mesenchymal stem cell (MSC)-like properties and some culture and expansion advantages. This population of MenSCs, compared to the broadly studied bone marrow derived stem cells (BM-MSCs) out-performs bone marrow derived mesenchymal stem cells in proliferation rate and support of hematopoietic stem cell (HSC) expansion in vitro. MenSCs demonstrate to maintain their stem cell properties over 2 years, being genetically stable, without expressing surface differentiation markers, and they also show the ability to differentiate into adipocytes, chondrocytes and osteoblast cells. MenSCs can be easily and periodically obtained, isolated and cultured.

### BACKGROUND OF THE INVENTION

Mesenchymal stem cell (MSC)-like are well known for exhibiting desirable properties in culture and expansion in vitro.
Particularly, bone marrow derived stem cells (BM-MSCs) are known for having the ability to support expansion in vitro of stem cells, such as hematopoietic stem cells (HSC).

HSC have to be expanded in vitro successfully in order to have enough quantities to be used for clinical research and for the treatment of a wide range of diseases. Nevertheless, these known MSC types used to support expansion of stem cells have a low availability and they are the main limiting factor when co-culturing cells.

Moreover, BM-MSCs cannot be easily and periodically obtained, isolated and cultured as requested, due to the restricted source.

Therefore, a high proliferation and a good differentiation of HSC is a key issue, but taking special attention on maintaining the expression of their stem cell markers (CD34+ and CD133+).

In the prior art, document US2007041948 has been described the co-culture of human endometrial stromal cell with HSC expressing CD34+, nevertheless, this method is uses endometrial stromal cells or epithelial cells obtained from fresh tissue, having a very limited availability for research and clinical purposes. The human endometrial stem cells are obtained by culturing finely cut fresh uterine tissue in a medium containing collagenase, the tissue is disaggregated, the cells are filtered and stromal cells are isolated, collecting the human endometrial stromal cells adhered to the cell culture dish.

WO1993018137 discloses the co-culture of HSC with stromal cells or stromal cells' conditioned media. The document describes HSC co-culture directly over a layer of stromal cells or alternatively, HSC are co-cultured separated from the stromal cells using a porous membrane. The aim of the invention is the growing of stem cells inhibiting the differentiation of the cells to improve their expansion and the culture of genetic modified human hematopoietic stem cells. The differentiation is reduced by using culture media supplemented with Leukemia Inhibitory Factor (LIF), where the inhibition of the differentiation also improves the integration of the construct.

WO2009/058356 and related application WO2013/025189 disclose methods for obtaining expanded human cord blood cells that may express CD34, SSEA-4, and HLA-II through co-culture with menstrual cells collected, isolated and cultured according to methods in WO2008/109063.

### SUMARY OF THE INVENTION

The present invention provides a method to obtain highly proliferative stem cell from menstrual fluid for clinical and research applications, including in vivo and in vitro cultures.

The MenSCs obtained using the method described in the present invention have culture and expansion advantages in the proliferation rate and in their ability for supporting hematopoietic stem cell (HSC) expansion in vitro.

In an embodiment of the invention, the method comprises the easy and periodically obtention, isolation and culture of MenSCs, where the MenSCs are obtained from menstrual blood.

In an embodiment of the invention, the method comprises obtaining MenSCs from menstrual blood, where the isolated MenSCs have high proliferation rates, determined at different time points of the culture. The proliferation rates can be compared to BM-MSCs isolated under standard conditions, as described below.

In an embodiment of the invention, the method provides an in vitro method for culturing menstrual stem cells (MenSCs) that exhibit mesenchymal stem cell (MSC)-like properties.

In an embodiment of the invention, the method comprises the selection of the MenSCs according to the immunophenotype based on mesenchymal and pluripotential stem cell markers.

In an embodiment of the invention, the method comprises the co-culture of MenSCs with cord blood stem cells in a growth factor-defined medium.

In an embodiment of the invention, the method comprises the ex vivo proliferation and support of HSCs.

In an embodiment of the invention, the method comprises the adipogenic, chondrogenic, and osteogenic differentiation of the MenSCs.

### BRIEF DESCRIPTION OF THE DRAWINGS

Figure 1 illustrates the menstrual blood-derived stem cells morphology and surface markers expression.
Figure 1(A). Morphology of MenSCs in culture, of freshly isolated menstrual stem cells (without passages or passage zero P0), after 2-week cell culture, and at the third passage (P3) showing a fibroblast-like spindle-shaped morphology. Scale bars 100 um.
Figure 1(B). Menstrual blood cells display stem cell-like phenotypic markers. In order to determine the immunophenotype MenSCs and BM-MSCs were stained by FITC, APC, PE or PECy7-conjugated antibodies against mesenchymal stromal, hematopoietic and pluripotential stem cell markers. Viable cells were determined through the stain LIVE/DEAD®Fixable dead cell kit and then analyzed by FC.
Figure 1(C). As shown in these figures, MenSCs display positive expression for mesenchymal stromal cells such as CD44, CD105, and CD73 while negative for CD14, CD34, CD45, HLA-DR, EPCAM, CD31, CD117, and CD271, and mildly positive for CD146. In addition, FC analysis showed that MenSCs do not express pluripotent surface markers such as SSEA-3, SSEA-4, and TRA-1-60.
   Abbreviations: MenSCs, menstrual blood-derived stem cells; BM-MSCs, bone marrow mesenchymal stem cells; FC, flow cytometry.
Figure 2 illustrates that menstrual blood-derived stem cells display high proliferation potential and CFU-F capacity.
Figure 2(A). MenSCs show high proliferation potential. In order to determine the proliferation potential, cells were cultured at 1000 cells/cm² in a 24-well plate and the cellular mitochondrial dehydrogenase was quantified at different time points. Starting from 6-days post-culture, MenSCs showed significantly high proliferation kinetics when compared to BM-MSCs.
Figure 2(B). CFU-F capacity. Serial dilutions of a defined number of cells were cultured and their potential for the formation of CFU was evaluated. Only colonies formed by more than 50 fibroblast-like cells were assessed. MenSCs significantly generated more CFUs than BM-MSCs.
Figure 2(C). Relative CFU-F capacity. After 21 days of cell culture, colonies were evaluated according to crystal violet staining (absorbance, 620 nm) with the purpose of reflecting their potential growing. MenSCs showed similar colonies size than BM-MSC. Bars represent the SE of triplicate (*p ≤ 0.05). Abbreviations: CFU-F, colonies forming unit of fibroblast; SE, standard error.
Figure 3 illustrates that menstrual blood-derived stem cells differentiate into mesodermal tissues. MenSCs have the ability to differentiate into adipose, bone and chondrocytes tissues after culture under appropriate differentiation media at varying degrees compared with BM-MSCs.
Figure 3(A). Adipocytic differentiation. Staining with oil red O for fat vacuoles showed lowest adipogenic differentiation in MenSCs respect to BM-MSC.
Figure 3(B). Osteocytic differentiation. Staining with alizarin red S for calcium deposits showed high differential potential of MenSCs compare to BM-MSCs.
Figure 3(C). Condrocytic differentiation. MenSCs stain positive with safranina O for sulfated proteoglycans.

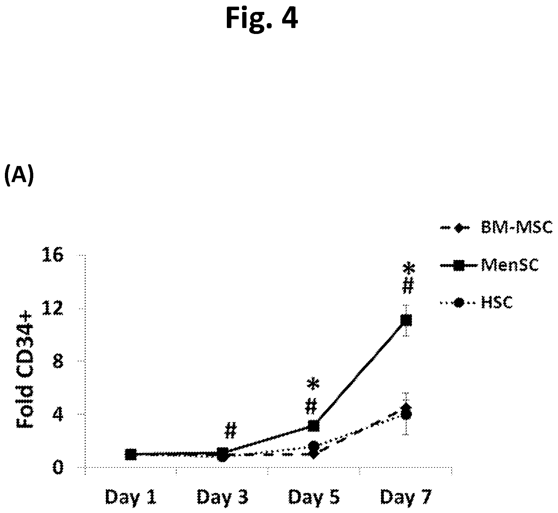

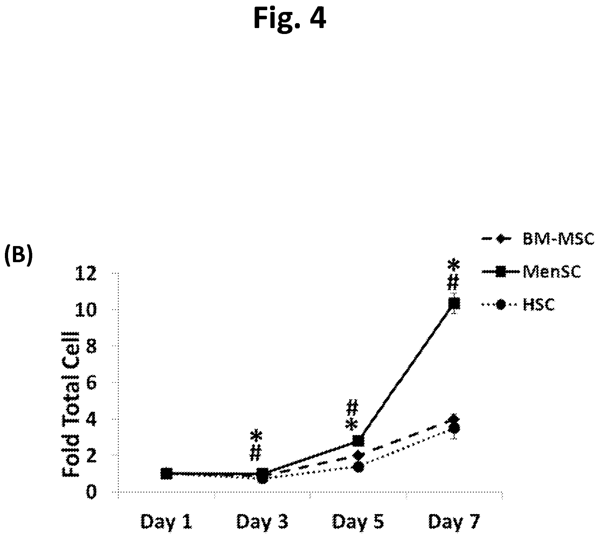

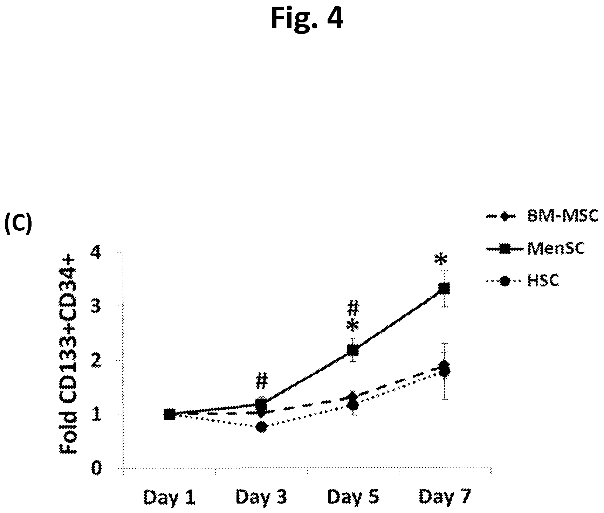

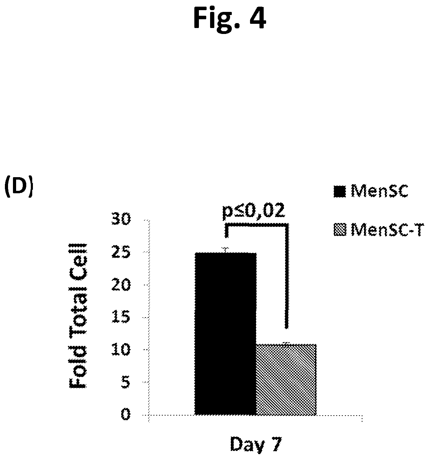

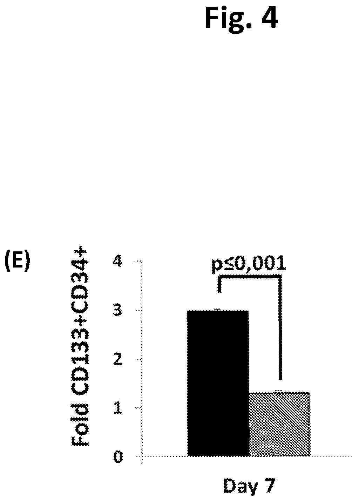

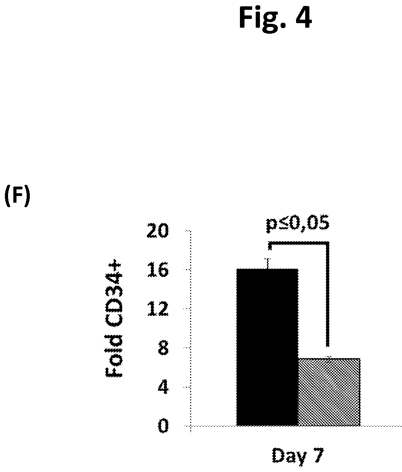

Figure 4 illustrates that MenSCs efficiently support the proliferation of CD34+ CD133+ hematopoietic stem cells. Cord blood CD34+CD133+ cells were immunomagneticaly isolated using CD34 beads, then cultured alone or co-cultured at a ratio of 1:4 to 1:5 in two different feeder layers from BM-MSCs and MenSCs, or without feeder (alone). Total number of cells and the expression of CD34+ CD133+ were evaluated at different time point during the course of the culture. Starting from 3 days post-culture, a statistically significant difference in favor of the co-culture with MenSCs was observed. By the end of the culture, a difference about 3 folds was observed when compared with cells cultured alone or with BM-MSCs.
Figure 4(A). Expansion of CD34+ cells cultured on feeder layers from BM.MSCs and MenSCs, or alone.
Figure 4(B). Total cells numbers fold increase compared to initial number of cells.
Figure 4(C). Expansion of CD34+CD133+ cells cultured on feeder layers from BM-MSCs and MenSCs, or alone. Bars represent the SE of triplicate (*p ≤ 0.05).
Figure 4 (D and E). Illustrates the co-culture of CD34+CD133+ HSC with MenSCs under direct contact (MenSC) and non-contact conditions (MenSC-T), using a separating membrane, Transwell® (T). CD34+CD133+ were cultured under similar media condition with the difference of the presence or absence of the separating membrane, Transwell®.
Figure 4(D) shows histogram representing the fold increase of total number of cells with or without the Transwell.
Figure 4(E) shows the fold increase of CD34+CD133+ cells population.
Figure 4(F) illustrates the fold increase of CD34+ cells population.

### DETAILED DESCRIPTION OF THE INVENTION

The following detailed description does not limit the scope of the invention to the examples and analysis described. While embodiments of the invention may be described, modifications, adaptations, and other implementations are possible. For example, substitutions, additions, or modifications may be made to the steps, equipments and compounds used in the methods described herein and may be modified by substituting, reordering, or adding stages equipments and compounds to the disclosed embodiments.

While stages and methods are described in terms of "comprising" various compounds or steps, the methods can also consist of more steps or using more equipments and compounds, unless stated otherwise. Additionally, the terms "a," "an," and "the" are intended to include plural alternatives, unless stated otherwise specified.

The mesenchymal stem cells obtained according to the present invention can be harvested from menstrual blood, which is a clear difference regarding the invasive procedures and the ethical controversy compared to embryonic stem cells related procedures.

MenSCs display stem cell-like phenotypic markers and show great potential for self-renewal, plasticity, and undifferentiated proliferation for long periods of time in vitro. A large number of studies have shown that MenSCs are a population of pluripotent cells.

Due to many limiting factors related to their practical usage in routine applications as detailed in the introductory section, there is an urge of creating a more accessible "product" that can fill the gap and as well as the supply and demand of an expanding stem cell therapy field. Supported by recently published data and our results, the present invention positions the cells isolated from menstrual fluids on the top of the list of cells candidates to fill up that clinical need.

Several lines of MenSCs associated with novel regenerative properties have been isolated. Menstrual cells have been collected, isolated and cultured according to classic methods as described by Meng et al. 2007 and document WO2008/109063.

Those methods disclose the isolation and processing of menstrual cells, where isolation may be performed with at least one of the operations of centrifugation, density gradient sedimentation or filtration. The processing stages to obtain the menstrual cell include the steps of obtain, isolate and preserve the menstrual cells where menstrual cells can be used later fresh or cryopreserved.

Thus, those processes show a source of stem cells, where viable menstrual cells are prepared for cryopreservation and are stored at cell banks being a more available source than cord blood cells, that is easily accessible, highly expandable in vitro and may be an alternative for autologous or heterologous stem cell applications.

### Characterization of the MenSCs

A detailed characterization of the MenSCs has been made for a complete comparison with related cells from other sources in order to allow the evaluation and comparative analysis of the advantages of MenSCs and also their safety/efficacy profile for clinical applications.

### 1. Morphology

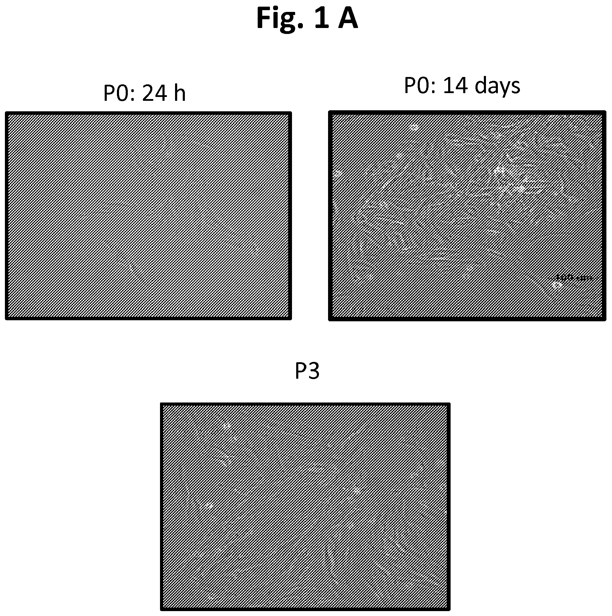

The menstrual blood-derived stem cells morphology in culture is shown in Figure 1(A), having a fibroblast-like spindle-shaped morphology.

The morphology is maintained during the successive culture passages, and can be observed in freshly isolated menstrual stem cells (without passages or passage zero), after 24 hours of culture and after 2-week cell culture and a confluent culture at the third passage.

### 2. Immunophenotype

In the present invention, it has been completed the full phenotypical and functional characterization of the MenSCs, while concentrating on their yet unexplored properties for the support of hematopoietic stem cells (CD34+ and CD133+) population.

MenSCs have been shown to be positive for mesenchymal stem cell markers including CD9, CD29, CD105, and CD73, and negative for hematopoietic markers such as CD34, CD45 and CD133, as expected for those cell types.

However, some groups have reported positive expression of embryonic markers such as SSEA-4 and Nanog in MenSCs, which were not found to be present on MSCs from other sources. This raises the question whether these cells presenting earlier markers of stemness represent a more primitive progenitor than MSCs from other sources, although there was a second group showing a different expression pattern in cells isolated and cultured under comparable conditions* (Meng et al. 2007).

It was analyzed an exhaustive list of mesenchymal phenotypic markers on menstrual stem cells (MenSCs) from 3 different donors and they were compared to the phenotypic markers on MSCs isolated from the bone marrow (BM-MSCs). Additionally, these cells were further characterized, using a large battery of markers looking not only for mesenchymal and embryonic markers, but also for endothelial and epithelial markers, as other cell types might represent a source of contamination of the MenSC culture.

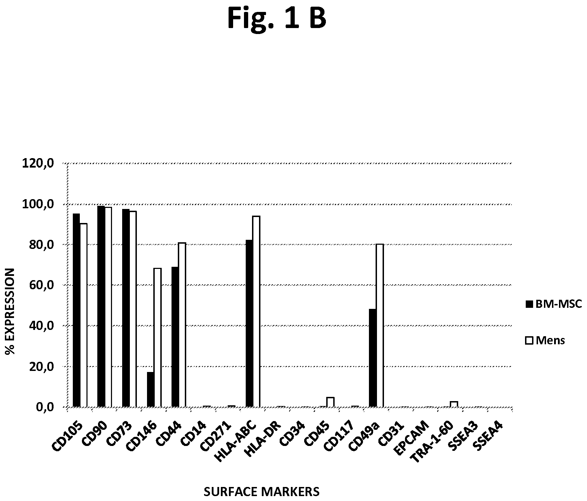

These quality-control parameters are essential when comparing similar cells from different sources as shown in Figure 1(B). For this analysis, MenSCs and BM-MSCs were stained by FITC, APC, PE or PECy7-conjugated antibodies against mesenchymal stromal, hematopoietic and pluripotential stem cell markers. Viable cells were determined through the stain LIVE/DEAD® Fixable dead cell kit and then analyzed by flow cytometry, FC.

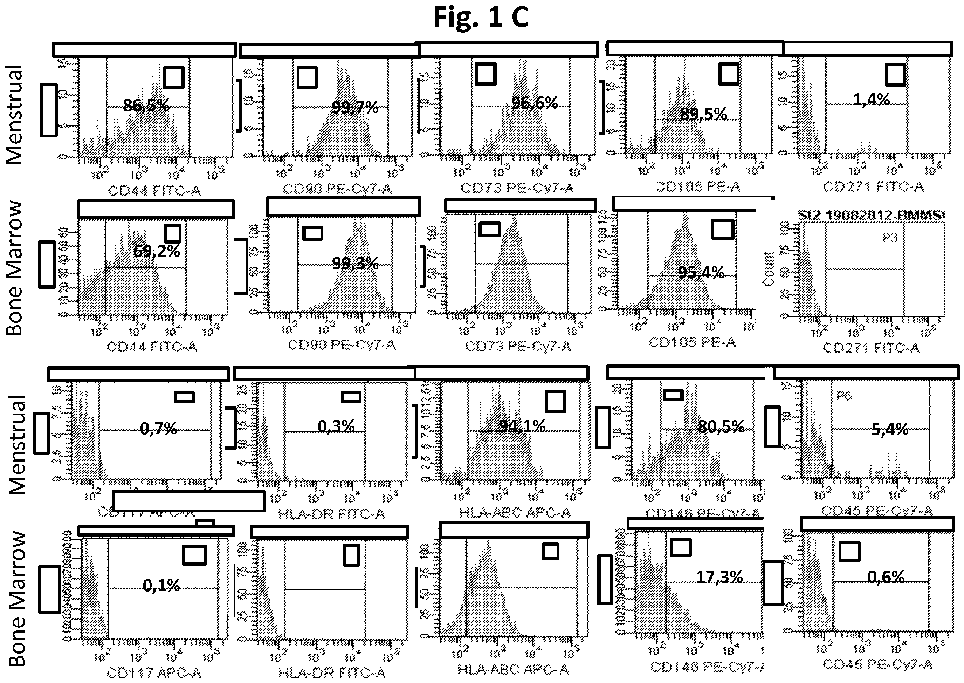

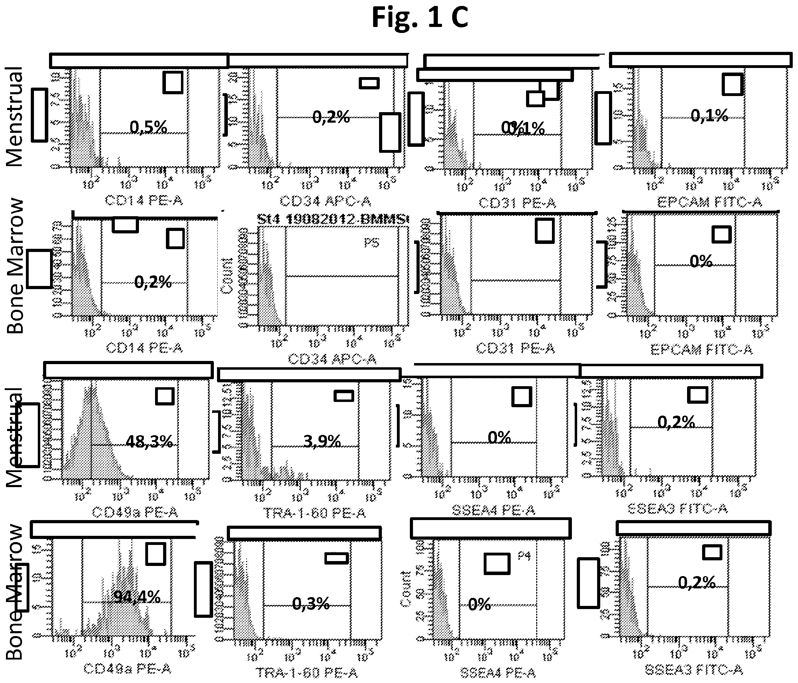

As shown in Figure 1(C), MenSCs display positive expression for mesenchymal stromal cells such as CD44, CD105, and CD73 while negative for CD14, CD34, CD45, HLA-DR, EPCAM, CD31, CD117, and CD271, and mildly positive expression for CD146.

In addition, flow cytometry analysis showed that MenSCs do not express pluripotent surface markers such as SSEA-3, SSEA-4, and TRA-1-60.

### 3. Clonogenicity and precursor frequency

The frequency of stem cell precursors, defined by their ability to form a cell colony *in vitro* (colony-forming unit, CFU) is another important property when characterizing a new stem cell source.

While MSCs can be virtually isolated from many organs, their low frequency or lack of clonogenicity can be a limiting factor for their clinical application. For instance, MSCs isolated from umbilical cord blood have comparable differentiation properties as cells isolated from bone marrow (BM-MSC); however, these cells are found at a much lower rate than in the later case, hampering their therapeutically usage.

It is thus essential to determine if MenSC possess a high or a low precursor frequency in comparison to MSCs isolated from other known sources. For this purpose cells were cultured at different serial dilution to determine their CFU-fibroblast frequency in comparison to BM-MSCs.

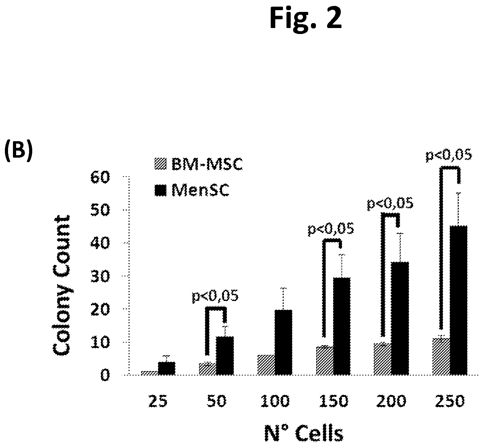

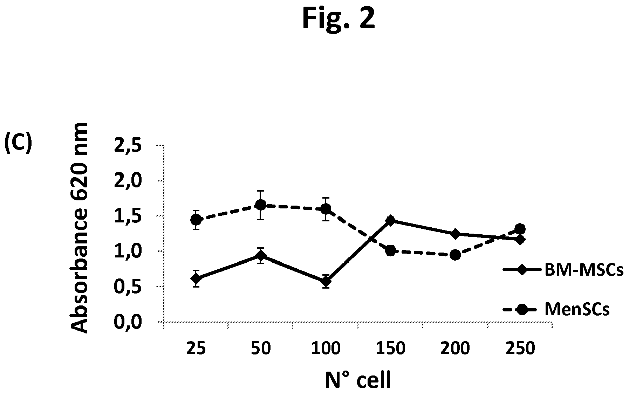

As shown in Figure 2 (B), at 21 day post-culture, it was noted a significantly higher number of CFUs where the MenSCs were cultured indicating a higher frequency of stem/progenitor cells.

### 4. Proliferation

In addition, MenSCs have shown a higher proliferation rate compared to other sources, with doubling times of MenSCs within 18-24 hr depending on culture technique and media used (Meng et al., 2007).

A good proliferation rate is essential for feasibility of clinical applications since stem cell-based therapies are usually efficient starting at a specific dose.

In most human clinical trials, 1 million-cells/kg appears to be the dose of choice; however, when allogenic or repeated usage seems possible, escalating the yield of cultures can become of utmost importance.

Nonetheless, a high proliferation is a two-edged sword that could also lead to genetic instability or the exhaustion of a specific stem cell pool. In fact, these MenSCs have been largely expanded *in vitro* without any mutation or visible abnormality at the chromosomal level. They maintained a telomerase activity greater than 50% even at passage 12 compared with human embryonic stem cells (Patel et al., 2008), and also appear to mildly express the chemokine receptor CXCR4 and the respective receptor for stromal cell-derived factor-1 (SDF-1), which play a significant role in the mediation of mesenchymal stem cell migration (Patel et al., 2008).

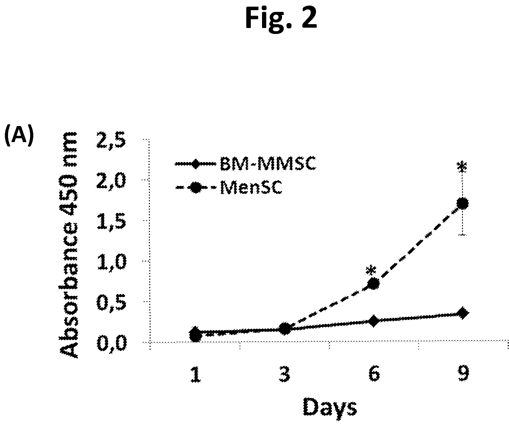

More interestingly, according to the present invention, these MenSCs cells did not show any sign of stem cell exhaustion evidenced by a steady expression of all stromal stem markers, a stable proliferation rate and CFU potential when compared as shown in Figure 2 (A). Such a high proliferative rate in the face of genetic stability and apparent preservation of multipotency indicates MenSCs as a type of stem cell that could present unexpected therapeutic properties, a fact that is also implied by their extensive differentiation capabilities.

### 5. Differentiation potential and regenerative properties

The ability of MenSCs to differentiate into adipose, bone, cartilage, cardiac, neural, hepatic and pancreatic cell types has been shown using standard differentiation techniques and media conditions.

A recent study by (Hida et al., 2008)* using co-culture with fetal mouse cardiomyocytes went still further, evidencing immortalization mediated by human telomerase reverse transcriptase (hTERT) on MenSCs (Hida et al., 2008). They also demonstrated that spontaneous beating can occur upon cardiogenic differentiation.

In addition, these multipotent cells have the ability to differentiate into various functional types, including respiratory epithelial cells, neurocytes, myocytes, endothelial cells, pancreatic cells, hepatocytes, adipocytes and osteocytes (Meng et al., 2007).

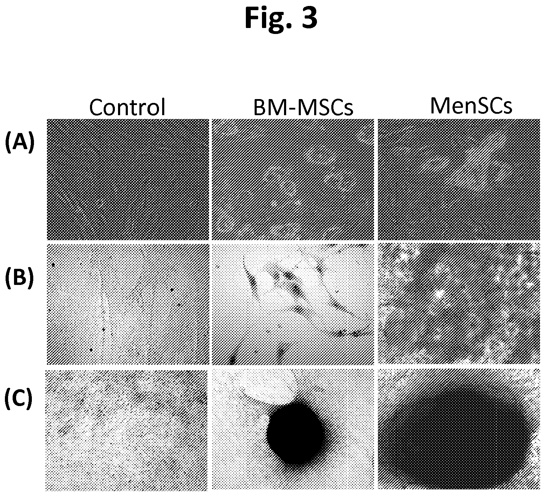

For the present invention it has been investigated the mesodermal differentiation potential of MenSCs and has been compared to BM-MSCs. MenSCs show the ability to differentiate into adipose, bone and chondrocytes tissues after culture under appropriate differentiation media at varying degrees compared with BM-MSCs. They showed a lower adipogenic differentiation, an equal osteocytic differentiation and a higher chondrocytic differentiation as shown in the different staining as shown in Figure 3.

### 6. Role of MenSCs in the support of hematopoiesis

The supportive effect of MSCs on hematopoiesis has been well documented in many studies including a published work of the inventor and a patent application Khoury et al, 2011 and patent application WO2010/138873, where expanding human hematopoietic stem cells (HSCs) to increase their numbers while maintaining their stem cell properties has therefore become an important area of research.

MSCs isolated from the bone marrow (BM-MSCs) have been used as a support for HSC expansion *in vitro* in preclinical and clinical settings. However, the ability of MenSCs to support hematopoietic stem cells *in vitro* and *in vivo* has not been established yet. Thus, their usefulness in supporting hematopoietic stem cell engraftment is not as clear as with BM-MSCs.

CD34+ CD133+ cells were immunomagneticaly isolated from human umbilical cords using CD34 beads, then cultured alone or co-cultured at a ratio of 1:4 to1:5 in different feeder layers from BM-MSCs and MenSCs. Total number of cells and the expression of CD34+ CD133+ were evaluated at different time point during the course of the culture. Starting from 3 days post-culture, a statistically significant different in favor of the co-culture with MenSCs was observed. By the end of the culture, a difference about 4 fold was observed for total cell numbers when compared with cells cultured alone or with BM-MSCs.

More interestingly, it was observed a significant higher expansion of CD34+ CD133+ cells cultured on feeder layers from BM-MSCs, MenSCs, or alone as shown in Figure 4 (C).

The results clearly demonstrate that HSCs co-cultured with MenSCs in a growth factor-defined medium, show a high proliferation rate while maintaining their stem cell markers (CD34+ and CD133+).

Compared with BM-MSCs, MenSCs elicit a more precocious early stem/progenitor formation and faster proliferation and differentiation of hematopoietic progenitors. This property to support hematopoiesis *in vitro* provides the rationale for their use in supporting hematopoietic reconstitution in clinical settings. The mechanism behind this superior yield is currently under investigation in our laboratory.

### 7. Role of direct contact of MenSCs in the co-culture

It is also important to take into consideration parameters such as safety issues, in order to avoid any undesired immune reaction. This issue can be addressed by isolating the supporting MenSCs from the culture used as supporting layer.

To determine whether the cell to cell contact is essential for the expansion of the CD34+ CD133+ population, the HSCs were co-cultured in the presence of MenSCs, in similar conditions, with the difference of the presence or absence of a separation membrane, denoted as T (Transwell®, Corning).

At day 7 of the expansion, cells were harvested, counted and then stained for the analysis of the different surface markers and analyzed by flow cytometry.

Expansion of HSC and HPC was significantly higher under MSC contact respect to noncontact growth conditions

### 8. Cell Procurement and Processing

Menstrual blood is collected from a healthy female subject during the earliest days of a menstrual cycle. Samples are collected in a menstrual silicone cup. The cells are harvested with the informed consent of the donor as approved by an Institutional Review Board of Universidad de los Andes. Menstrual blood samples are transferred into a tube with phosphate buffered saline (PBS) containing amphotericin B, penicillin, streptomycin and EDTA.

Menstrual blood mononuclear cells are separated by Ficoll-Paque density gradient and washed in PBS. Cells were subsequently cultured in a flask containing Dulbecco's modified Eagle's medium (DMEM) high Glucose supplemented with % penicillin/streptomycin, amphotericin B, glutamine and FBS at 35-40°C, using 2-10% CO2 in order to obtain adherent cells. Media were changed the next day to wash non adherent cells. Cells were replated as follows: adherent cells were detached using Trypsin-Edta, counted and sub-cultured. Afterwards, these cells were used as menstrual blood-derived stem cells (MenSCs).

Bone marrow mesenchymal stem cells (BM-MSCs) were maintained in the same medium. Hematopoietic Stem Cells (HSCs) were collected from fresh umbilical cord blood after informed consent. Mononuclear cell were isolated after centrifugation on Ficoll-Paque. CD34+ cells were enriched with a CD34 Positive Selection Kit. Labeled cells are separated using a magnet separation kit.

All the co-cultures are performed using MenSCs of different donors and BM-MSCs at early passages, no higher than 6 passages.

### 9. Adipogenic Differentiation

MenSCs and BM-MSCs are seed in adipogenic differentiation medium: complete DMEM media, dexamethasone, 3-isobutyl-1-methyl-xanthine (IBMX), recombinant human insulin, and indomethacin; and cultured for up 21 days with media changes every 3-4 days.

Control cells are cultured in completed DMEM media. Cells were subsequently stained with Oil Red O.

### 10. Osteogenic Differentiation

Cells are seed in osteogenic differentiation medium: complete DMEM media, dexamethasone, b-glycerophosphate, and ascorbate; and cultured for up 21 days with media changes every 3-4 days. Control cells were cultured in completed DMEM media. Cells are later stained with Alizarin.

### 11. Chondrogenic Differentiation

Cells are seed in chondrogenic differentiation medium: complete DMEM media, dexamethasone, b-glycerophosphate, and ascorbate; and cultured for up 21 days with media changes every 3-4 days. Control cells are cultured in completed DMEM media and subsequently stained with Safranina O.

### 12. Proliferation Assay

MenSCs and BM-MSCs are cultured in a 24-well plate in complete DMEM media. Cell proliferation and viability is determined at different days measuring cellular mitochondrial dehydrogenase and quantified by spectrophotometry using absorbance 440 nm.

Various independent experiments are performed in triplicate. The Student's *t-*test was used to calculate statistical difference. Data with a *p* value of *p* ≤ 0.05 is considered to be statistically significant.

### 13. Colony Forming Unit (CFU) Assay

In order to quantify functional mesenchymal stem cells, MenSCs and BM-MSCs were evaluated for frequency of Colony-Forming Unit-Fibroblast (CFU-F). CFU-F were evaluated plating between 25-250 cells per well of a 6-well plate at five different densities in complete DMEM media, in triplicates.

After 21 days, cells were stained with crystal violet in diluted methanol. After six washes, colonies formed by more than 50 fibroblast-like cells were counted under light microscope at low magnification. Results were expressed as CFU/number of cells plated. Then, cell-incorporated crystal violet was solubilized by incubation with diluted acetic acid and quantified by spectrophotometry at absorbance, 620 nm. The Student's *t*-test was used to calculate statistical difference. Data with a *p* value of *p* ≤ 0.05 was considered to be statistically significant.

### 14. Immunophenotypic Characterization of MenSC

For cell surface antigen analysis, cells were harvested, washed with cytometer buffer (PBS + BSA + Sodium Azide) and incubated with the specific antibody for 20 min a 4°C in cytometer buffer containing the specific fluorescein isothiocynate (FITC), allophycocyanin (APC), phycoerythrin (PE), or phycoerythrin with cyanin-7 (PECy7) labeled antibodies. Antibodies for human cell surface antigens CD14, CD44, CD90, CD271, CD105, CD73, CD117, CD45, CD34, HLA-ABC, HLA-DR, CD146, EPCAM, CD31, TRA-1-60, CD49a, SSEA3, SSEA4, CD133 and CD3 were used.

In the analysis, the corresponding isotype-matched antibodies are used as negative controls. In addition, a dead cell stain kits is used to determine the viability cells by flow cytometry. Data is collected and analyzed using FACS software.

### In vitro expansion of Cord Blood CD34+ CD133+ cells

Confluent layers of MenSCs and BM-MSCs were irradiated (15Gy). CD34+ CD133+ hematopoietic stem cells are then co-cultured with feeder layers using StemSpan medium supplemented with human growth factors: FGF-1, SCF, TPO, IGFBP2, heparin and penicillin/streptomycin.

Briefly, CD34+ CD133+cells are plated directly on the feeder layer or in Transwell above the feeder layer (noncontact growth conditions) in an appropriate ratio in growth factor-supplemented StemSpan media in 24-well plates. The medium was half changed every day and additionally supplemented. All the expanded cord blood cells were harvested after different days by vigorous pipetting, washed in PBS, and the cells total number were determined by trypan blue.

In addition, cells were stained with CD3-APC, CD45-FITC, CD34-PECy7, CD133-PE, and a dead cell stain kit. Expression of surface markers was then analyzed by flow cytometry. Various independent analyses are performed. The Student's *t*-test was used to calculate statistical difference. Data with a *p* value of *p* ≤ 0.05 is considered to be statically significant.

### 15. Expansion of CD34+CD133+ cells on MenSCs.

MSCs were seeded in 24 well plate and cultured on MenSC media prior to the coculture. MenSC media consists of DMEM-high glucose supplemented with FBS, penicillin streptomycin, amphotericin B and L-glutamine. The assays were preformed in triplicate. A fourth well was seeded for the cell quantification before radiation procedure. At day 1, once the MSCs cultures are in confluent layers, the cells were irradiated (15 Gy).

HSCs CD34+CD133+ cells derived from umbilical cord blood were obtained using CD34 immunomagnetic purification. The HCS cells were thawed as follows:
The cryotube was placed in a 30-40°C bath and cold DMEM media is slowly added to the tube (without using FBS). The cells were collected when adding the DMEM cold media. This procedure is repeated until recovering all the cells. Then, the mixture is centrifugated at room temperature. The supernatant is removed, resuspending the pellet on DMEM without supplemented FBS. Cells are quantified after adding trypan blue.

HSCs are co-cultured over adherent monolayer MSCs at 1:4 ratio of HSC:MSC cells /well in a 24 well plat. The culture was feed with supplemented StemSpan® media. Each following day the media was fed removing the residual media, and then adding fresh media.

The quantification of the HSCs cells is performed after resuspending the cells after carefully pipetting 10 times avoiding the disturbance of the MSCs layer. The HSCs cells are analyzed by FACS.

The analysis is made on different days for FITC, APC, PE, PECy7, CD45, CD3, CD133, CD34 antibodies.

### EXAMPLES

### EXAMPLE 1

### Cell Procurement and Processing

Menstrual blood was collected in a menstrual silicone cup (Mialuna®, Santiago, Chile). Menstrual blood samples were transferred into a 50 ml tube with 10 ml phosphate buffered saline (PBS) containing 0.25 mg/mL amphotericin B (Gibco, Paisley, UK), penicillin 100 IU, streptomycin 100 mg/mL and 2 mM EDTA (all from Sigma, USA).

Menstrual blood mononuclear cells were separated by Ficoll-Paque (Sigma-Aldrich, St Lois, USA) (1.077 g/ml) density gradient according to the manufacturer's instructions and washed in PBS. Cells were subsequently cultured in a T25 flask (Falcon®, Becton Dickinson) containing Dulbecco's modified Eagle's medium (DMEM) high Glucose (Gibco, Paisley, UK) supplemented with 1% penicillin/streptomycin, 1% amphotericin B, 1% glutamine (Gibco, Paisley, UK) and 15% FBS (Lonza, Walkersville, MD USA) at 37°C, 5% CO2 in order to obtain adherent cells.

Media were changed the next day to wash non adherent cells. Cells were replated as follows: adherent cells were detached using 0.05% Trypsin-Edta (Gibco, UK), counted and sub-cultured. Afterwards, these cells were used as menstrual blood-derived stem cells (MenSCs).

The cocultures were performed using MenSCs of 3 different donors and BM-MSCs at early passages (2 to 6 passages) using bone marrow mesenchymal stem cells (BM-MSCs) gifts from L. Salazar, Laboratorio Clínico, Cells for Cells, Chile; Hematopoietic Stem Cells (HSCs); and Mononuclear cell isolated after centrifugation on Ficoll-Paque. CD34+ cells were enriched with EasySep™ Human Cord Blood CD34 Positive Selection Kit (Stem Cell Technologies, Vancouver, Canada) according to the manufacturer's instructions. Labeled cells are separated using an EasySep™ magnet (Stem Cell Technologies).

### EXAMPLE 2

### Adipogenic Differentiation

MenSCs and BM-MSCs were seeded at a concentration of 1,5x10E4 cells/cm2 in adipogenic differentiation medium: complete DMEM media, 1mM dexamethasone, 0.5 mM 3-isobutyl-1-methyl-xanthine (IBMX), 10ug/ml recombinant human insulin, and 100 mM indomethacin; and cultured for up 21 days with media changes every 3-4 days.

Control cells were cultured in completed DMEM media. Cells were subsequently stained with Oil Red O (Sigma-Aldrich, St Lois, USA).

### Osteogenic Differentiation

Cells were seeded at a concentration of 2x10E4 cells/cm2 in osteogenic differentiation medium: complete DMEM media, 100nM dexamethasone, 10mM b-glycerophosphate, and 0.2MM ascorbate; and cultured for up 21 days with media changes every 3-4 days.

Control cells were cultured in completed DMEM media. Cells were stained with Alizarin Red (Sigma-Aldrich, St Lois, USA).

### Chondrogenic Differentiation

Cells were seeded at a concentration of 3x10E4 cells/10uL in chondrogenic differentiation medium: complete DMEM media, 100nM dexamethasone, 10mM b-glycerophosphate, and 0.2mM ascorbate; and cultured for up 21 days with media changes every 3-4 days.

Control cells were cultured in completed DMEM media. Cells were subsequently stained with Safranina O (Sigma-Aldrich, St Lois, USA).

### EXAMPLE 3

### Proliferation Assay

MenSCs and BM-MSCs were cultured at 1000 cells/cm2 in a 24-well plate (Falcon®, Becton Dickinson) in complete DMEM media.

Cell proliferation and viability were determined at days 3, 6 and 9 measuring cellular mitochondrial dehydrogenase by Quick Cell Proliferation Assay Kit II (BioVision, Milpitas, CA USA) according to the manufacturer's instructions and quantified spectrophotometrically (absorbance, 440 nm).

Three independent experiments were performed in triplicate

### EXAMPLE 4

### Colony Forming Unit (CFU) Assay

In order to quantify functional mesenchymal stem cells, MenSCs and BM-MSCs were evaluated for frequency of Colony-Forming Unit-Fibroblast (CFU-F). CFU-F were evaluated plating between 25-250 cells per well of a 6-well plate (Falcon®, Becton Dickinson) at five different densities in complete DMEM media, in triplicates.

After 21 days, cells were stained with 0.5% crystal violet (Sigma-Aldrich, St.Louis, MO) in 10% methanol for 20 minutes. After six washes, colonies formed by more than 50 fibroblast-like cells were counted under light microscope at low magnification.

Results were expressed as CFU/number of cells plated. Then, cell-incorporated crystal violet was solubilized by incubation with acetic acid 33% and quantified spectrophotometrically (absorbance, 620 nm).

### EXAMPLE 5

### Immunophenotypic Characterization of MenSC

For cell surface antigen analysis, cells were harvested, washed with cytometer buffer (PBS + 0.2% BSA + 0.01% Sodium Azide [all from Sigma-Aldrich, St Lois, USA]) and incubated with the specific antibody at concentrations recommended by the respective manufacturer for 20 min a 4°C in cytometer buffer containing the specific fluorescein isothiocynate (FITC), allophycocyanin (APC), phycoerythrin (PE), or phycoerythrin with cyanin-7 (PECy7) labeled antibodies.

Antibodies for human cell surface antigens CD14, CD44, CD90, CD271, CD105, CD73, CD117, CD45, CD34, HLA-ABC, HLA-DR, CD146, EPCAM, CD31, TRA-1-60, CD49a, SSEA3, SSEA4, CD133 and CD3 were purchase from BD Pharmingen™ (BD Biosciences, San Jose, CA), R&D System (Minneapolis, MN, USA) and Biolegend (San Diego, CA, USA).

In all experiments, the corresponding isotype-matched antibodies were used as negative controls. In addition, LIVE/DEAD®Fixable dead cell stain kits (Invitrogen) were used to determine the viability cells by flow cytometry according to the manufacturer's protocol. Data (10,000 events) were collected using a FACS Canto II Flow cytometer (BD Biosciences, San Jose, CA) and analyzed on FACS Diva software (BD Biosciences).

### EXAMPLE 6

### In vitro expansion of Cord Blood CD34+ CD133+ cells

Confluent layers of MenSCs and BM-MSCs were irradiated (15Gy) in the Chilean Commission for Nuclear Energy Facilities.

CD34+ CD133+ hematopoietic stem cells were then co-culture with these feeder layers using StemSpan (Stem Cell Technologies, Vancouver, Canada) supplemented with human growth factors: 10 ng/ml FGF-1, 10 ng/ml SCF, 20 ng/ml TPO, 100 ng/ml IGFBP2 -all R&D systems (Minneapolis, MN, USA)-, 100 micrograms/ml of heparin and 1 x penicillin/streptomycin (Gibco, Paisley, UK).

Briefly, CD34+ CD133+cells were plated directly on these feeder layer or in Transwell (0.4 µm pore size; BD Biosciences) above the feeder layer (noncontact growth conditions) in a 1:4-1:5 ratio in growth factor-supplemented StemSpan in a 24-well plates (Falcon®, Becton Dickinson), at 500 µl/well. The medium was half changed every day and additionally supplemented with 100 µl/well. All the expanded cord blood cells were harvested after 3, 5 and 7 days by vigorous pipetting, washed in PBS, and the cells total number were determined by trypan blue.

In addition, cells were stained with CD3-APC, CD45-FITC, CD34-PECy7, CD133-PE, and LIVE/DEAD®Fixable dead cell stain. Expression of surface markers was then analyzed by flow cytometry. Three independent experiments were performed in triplicate. The Student's t-test was used to calculate statistical difference. A p value of p ≤ 0.05 was considered to be statically significant.

### EXAMPLE 7

### Expansion of CD34+CD133+ cells on MenSCs.

MSCs were seeded in 24 well plate at a concentration of 5x10⁴cells/well. Cells were cultured on MenSC media prior to the coculture. MenSC media consists of DMEM-high glucose (Gibco #10313021) supplemented with 15% FBS, 1% penicillin streptomycin (Gibco #15140122), 1% amphotericin B (Biological #03-028-1B) and 1% L-glutamine (Gibco #25030-081). The assays were preformed in triplicate. A fourth well was seeded for the cell quantification before radiation procedure.

At day 1, once the MSCs cultures are in confluent layers, the cells were irradiated (15 Gy) in the Chilean Commission for Nuclear Energy Facilities, CCHEN.

HSCs CD34+CD133+ cells derived from umbilical cord blood were obtained using CD34 immunomagnetic purification. The HCS cells were thawed as follows:
The cryotube was placed for 10" in a 37°C bath and 1ml of cold DMEM media was slowly added to the tube (without using FBS). The cells were collected when adding the DMEM cold media. This procedure is repeated until recovering all the cells. Then, the mixture is centrifugated 5' at 1200rpm at room temperature. The supernatant is removed, resuspending the pellet on DMEM without supplemented FBS. Cells are quantified after adding trypan blue.

HSCs are cocultured over adherent monolayer MSCs at 1:4 and/or 1:5 ratio of HSC: MSC, using 10⁴ HSC cells and 4x10⁴ MSC cells /well in a 24 well plat. The culture was feed with supplemented StemSpan® media (StemCell Technologies #9610) 500uL/well (initial volume). Each following day the media was fed removing the residual media over 200ml, and then adding 100ml of fresh media.

The quantification of the HSCs cells is performed after resuspending the cells after carefully pipetting 10 times avoiding the disturbance of the MSCs layer. The HSCs cells are analyzed by FACS.

The analysis is made on days 3, 5 and 7 for FITC, APC, PE, PECy7, CD45, CD3, CD133, CD34 antibodies.

## Claims

1. A method for culturing and proliferating hematopoietic stem cells, comprising the steps of:
(a) culturing hematopoietic stem cells expressing CD34+ and CD133+;
(b) culturing menstrual stem cells obtained from a human donor during the earliest days of menstruation, said menstrual stem cells having positive expression for CD44, CD105 and CD73 and negative expression for CD14, CD34, CD45, HLA-DR, EPCAM, CD31, CD117, CD271, SSEA-3, SSEA-4 and TRA-1-60;
(c) co-culturing the hematopoietic stem cells cultured in step (a) and the menstrual stem cells cultured in step (b), whereby the hematopoietic stem cells proliferate.

2. The method of claim 1, wherein the cultured menstrual stem cells in step (b) are irradiated.

3. The method of claim 2, wherein the cultured menstrual stem cells are irradiated with 15Gy.

4. The method of claim 1, wherein the cultured menstrual stem cells in step (b) are cultured in supplemented DMEM-high glucose media.

5. The method of claim 4, wherein DMEM-high glucose media is supplemented with 15% FBS, 1 % penicillin streptomycin, 1 % amphotericin B and 1 % L-glutamine.

6. The method of claim 1, wherein the co-cultured cells of step (c) are cultured with supplemented culture media.

7. The method of claim 6, wherein the supplemented culture media is supplemented with FGF-1 , SCF, TPO, IGFBP2, heparin and penicillin/streptomycin.

8. The method of claim 7, wherein the supplemented media contains 10 ng/ml FGF-1, 10 ng/ml SCF, 20 ng/ml TPO, 100 ng/ml IGFBP2, 100 micrograms/ml of heparin and 1 x penicillin/streptomycin.

9. The method of claim 1, wherein prior to culturing in step (a) the hematopoietic stem cells are obtained by the steps of:
(i) collecting the hematopoietic stem cells from fresh umbilical cord blood;
(ii) concentrating cells using antibody complexes and dextran-coated magnetic particles; and
(iii) separating cells attached to the magnetic particles using high-gradient magnetic field.

10. The method according claim 9, wherein the antibodies antibody complexes are antibodies to CD 34 and CD133.

11. The method according claim 9, wherein the antibodies antibody complexes are antibodies to FITC, APC, PE, PECy7, CD45, CD3, CD133, CD34.

12. The method of claim 1, wherein the menstrual stem cells are obtained by the steps of:
(i) collecting the menstrual blood;
(ii) washing the menstrual cells by adding phosphate buffered saline;
(iii) separating the menstrual blood mononuclear cells through a Ficoll density gradient;
(iv) washing the menstrual blood mononuclear cells by adding phosphate buffered saline;
(v) culturing the menstrual blood mononuclear cells in supplemented media;
(vi) removing non adherent cells;
(vii) sub-culturing and cryo-preserving the adherent cells;
(viii) reacting the adherent cells with antibodies to human cell surface antigens; and.
(ix) isolating the cells bound to the antibody.

## Patentansprüche

1. Verfahren zur Kultivierung und Vermehrung von hämatopoetischen Stammzellen, umfassend die folgenden Schritte:
a) Kultivieren von hämatopoetischen Stammzellen, die CD34+ und CD133+ exprimieren;
b) Kultivieren von Menstruationsstammzellen, die von einem menschlichen Spender während der ersten Tage der Menstruation gewonnen werden, wobei die Menstruationsstammzellen eine positive Expression für CD44, CD105 und CD73 und eine negative Expression für CD14, CD34, CD45, HLA-DR, EPCAM, CD31, CD117, CD271, SSEA-3, SSEA-4 und TRA-1-60 aufweisen;
c) Cokultivieren der in Schritt (a) kultivierten hämatopoetischen Stammzellen und der in Schritt (b) kultivierten Menstruationsstammzellen, wodurch sich die hämatopoetischen Stammzellen vermehren.

2. Verfahren nach Anspruch 1, wobei die in Schritt (b) kultivierten Menstruationsstammzellen bestrahlt werden.

3. Verfahren nach Anspruch 2, wobei die kultivierten Menstruationsstammzellen mit 15 Gy bestrahlt werden.

4. Verfahren nach Anspruch 1, wobei die in Schritt (b) kultivierten Menstruationsstammzellen in einem supplementierten glucosereichen DMEM-Medium kultiviert werden.

5. Verfahren nach Anspruch 4, wobei das glucosereiche DMEM-Medium mit 15 % FBS, 1 % Penicillin/Streptomycin, 1 % Amphotericin B und 1 % L-Glutamin supplementiert ist.

6. Verfahren nach Anspruch 1, wobei die cokultivierten Zellen von Schritt (c) mit supplementiertem Kulturmedium kultiviert werden.

7. Verfahren nach Anspruch 6, wobei das supplementierte Kulturmedium mit FGF-1, SCF, TPO, IGFBP2, Heparin und Penicillin/Streptomycin supplementiert ist.

8. Verfahren nach Anspruch 7, wobei das supplementierte Medium 10 ng/ml FGF-1, 10 ng/ml SCF, 20 ng/ml TPO, 100 ng/ml IGFBP2, 100 Mikrogramm/ml Heparin und 1 x Penicillin/Streptomycin enthält.

9. Verfahren nach Anspruch 1, wobei die hämatopoetischen Stammzellen vor dem Kultivieren in Schritt (a) durch die folgenden Schritte gewonnen werden:
i. Sammeln der hämatopoetischen Stammzellen aus frischem Nabelschnurblut;
ii. Konzentrieren der Zellen mithilfe von Antikörperkomplexen und dextranbeschichteten magnetischen Partikeln; und
iii. Abtrennen der an den magnetischen Partikeln anhaftenden Zellen mittels eines hochgradienten Magnetfelds.

10. Verfahren nach Anspruch 9, wobei die Antikörper Antikörperkomplexen Antikörper gegen CD34 und CD133 sind.

11. Verfahren nach Anspruch 9, wobei die Antikörper Antikörperkomplexen Antikörper gegen FITC, APC, PE, PECy7, CD45, CD3, CD133, CD34 sind.

12. Verfahren nach Anspruch 1, wobei die Menstruationsstammzellen durch die folgenden Schritte gewonnen werden:
i. Sammeln des Menstruationsbluts;
ii. Waschen der Menstruationszellen durch Zugabe von phosphatgepufferter Kochsalzlösung;
iii. Abtrennen der mononuklearen Zellen des Menstruationsbluts durch einen Ficoll-Dichtegradienten;
iv. Waschen der mononuklearen Zellen des Menstruationsbluts durch Zugabe von phosphatgepufferter Kochsalzlösung;
v. Kultivieren der der mononuklearen Zellen des Menstruationsbluts in supplementiertem Medium;
vi. Entfernen von nicht adhärenten Zellen;
vii. Subkultivieren und Kryokonservieren der adhärenten Zellen;
viii. Reagierenlassen der adhärenten Zellen mit Antikörpern gegen menschliche Zelloberflächenantigene; und.
ix. Isolieren der an den Antikörper gebundenen Zellen.

## Revendications

1. Procédé de culture et de prolifération de cellules souches hématopoïétiques, comprenant les étapes de :
(a) culture de cellules souches hématopoïétiques exprimant les CD34+ et CD133+ ;
(b) culture de cellules souches de sang menstruel obtenues d'un donneur humain durant les tout premiers jours de la menstruation, lesdites cellules souches de sang menstruel présentant une expression positive pour les CD44, CD105 et CD73 et une expression négative pour les CD14, CD34, CD45, HLA-DR, EPCAM, CD31, CD117, CD271, SSEA-3, SSEA-4 et TRA-1-60 ;
(c) coculture des cellules souches hématopoïétiques cultivées dans l'étape (a) et des cellules souches de sang menstruel cultivées dans l'étape (b), selon lequel les cellules souches hématopoïétiques prolifèrent.

2. Procédé selon la revendication 1, dans lequel les cellules souches de sang menstruel cultivées dans l'étape (b) sont irradiées.

3. Procédé selon la revendication 2, dans lequel les cellules souches de sang menstruel cultivées sont irradiées avec 15 Gy.

4. Procédé selon la revendication 1, dans lequel les cellules souches de sang menstruel cultivées dans l'étape (b) sont cultivées dans du milieu DMEM riche en glucose complémenté.

5. Procédé selon la revendication 4, dans lequel le milieu DMEM riche en glucose est complémenté avec 15 % de FBS, 1 % de pénicilline/streptomycine, 1 % d'amphotéricine B et 1 % de L-glutamine.

6. Procédé selon la revendication 1, dans lequel les cellules cocultivées de l'étape (c) sont cultivées avec du milieu de culture complémenté.

7. Procédé selon la revendication 6, dans lequel le milieu de culture complémenté est complémenté avec du FGF-1, du SCF, de la TPO, de l'IGFBP2, de l'héparine et de la pénicilline/streptomycine.

8. Procédé selon la revendication 7, dans lequel le milieu complémenté contient 10 ng/ml de FGF-1, 10 ng/ml de SCF, 20 ng/ml de TPO, 100 ng/ml d'IGFBP2, 100 microgrammes/ml d'héparine et de la pénicilline/streptomycine 1X.

9. Procédé selon la revendication 1, dans lequel avant la culture dans l'étape (a) les cellules souches hématopoïétiques sont obtenues par les étapes de :
(i) prélèvement des cellules souches hématopoïétiques à partir de sang de cordon ombilical frais ;
(ii) concentration des cellules en utilisant des complexes d'anticorps et des particules magnétiques revêtues de dextrane ; et
(iii) séparation des cellules fixées aux particules magnétiques en utilisant un champ magnétique de gradient élevé.

10. Procédé selon la revendication 9, dans lequel les anticorps des complexes d'anticorps sont des anticorps dirigés contre les CD34 et CD133.

11. Procédé selon la revendication 9, dans lequel les anticorps des complexes d'anticorps sont des anticorps dirigés contre le FITC, l'APC, la PE, la PECy7, les CD45, CD3, CD133, CD34.

12. Procédé selon la revendication 1, dans lequel les cellules souches de sang menstruel sont obtenues par les étapes de :
(i) prélèvement du sang menstruel ;
(ii) lavage des cellules de sang menstruel par addition de solution tampon phosphate ;
(iii) séparation des cellules mononucléées de sang menstruel à travers un gradient de densité Ficoll ;
(iv) lavage des cellules mononucléées de sang menstruel par addition de solution tampon phosphate ;
(v) culture des cellules mononucléées de sang menstruel dans du milieu complémenté ;
(vi) élimination des cellules non adhérentes ;
(vii) repiquage et cryoconservation des cellules adhérentes ;
(viii) réaction des cellules adhérentes avec des anticorps dirigés contre des antigènes de surface cellulaire humains ; et
(ix) isolement des cellules liées à l'anticorps.
